Europäisches Patentamt

European Patent Office

Office européen des brevets

(19)

(11) Veröffentlichungsnummer: **0 261 524**
**A2**

(12) **EUROPÄISCHE PATENTANMELDUNG**

(21) Anmeldenummer: 87113312.0

(22) Anmeldetag: **11.09.87**

(51) Int. Cl.⁴: **A61K 31/24** ,
//(A61K31/24,31:10)

(30) Priorität: 24.09.86 DE 3632359

(43) Veröffentlichungstag der Anmeldung:
**30.03.88 Patentblatt 88/13**

(84) Benannte Vertragsstaaten:
**AT BE CH DE ES FR GB GR IT LI NL SE**

(71) Anmelder: **Troponwerke GmbH & Co. KG**
**Berliner Strasse 156**
**D-5000 Köln 80(DE)**

(72) Erfinder: **Beckermann, Bernhard, Dr.**
**Langemarckweg 56**
**D-5060 Bergisch-Gladbach 2(DE)**
Erfinder: **Dell, Hans-Dieter, Dr.**
**Kalmüntener Strasse 5**
**D-5060 Bergisch-Gladbach 2(DE)**
Erfinder: **Horstmann, Harald, Dr.**
**Claudiusweg 19**
**D-5600 Wuppertal 1(DE)**
Erfinder: **Kraus, Reinhold**
**Pfaffendorfstrasse 77**
**D-5000 Koeln 91(DE)**

(74) Vertreter: **Mann, Volker, Dr. et al**
**c/o Bayer AG Konzernverwaltung RP**
**Patentabteilung**
**D-5090 Leverkusen 1, Bayerwerk(DE)**

(54) **Arzneimittelzubereitungen.**

(57) Arzneimittelzubereitungen enthaltend 2-(2-Hydroxyethoxy)-ethyl-N-($\alpha,\alpha,\alpha$-trifluor-m-tolyl)-anthranilat und Dimethylsulfoxid.

EP 0 261 524 A2

## Arzneimittelzubereitungen

Die Erfindung betrifft Arzneimittelzubereitungen, die 2-(2-Hydroxy-ethoxy)-ethyl-N-($\alpha,\alpha,\alpha$-trifluor-m-tolyl)-anthranilat und Dimethylsulfoxid enthalten, Verfahren zu ihrer Herstellung und ihre Verwendung.

2-(2-Hydroxy-ethoxy)-ethyl-N-($\alpha,\alpha,\alpha$-trifluor-m-tolyl)-anthranilat, nach INN Etofenamat genannt, ist ein bekannter antiinflammatorisch und antiphlogistisch wirkender Wirkstoff, der besonders in Form von Gelen angewendet wird (Arzneimittelforschung 27 (I), Sonderheft 6b, 1299 bis 1364 (1977)).

Es ist ebenfalls bekannt, daß Dimethylsulfoxid (DMSO) in der Lage ist, die in ihm gelösten Arzneistoffe in kürzester Zeit durch die Haut in die Blutbahn zu schleusen (P. H. List, Arzneiformenlehre, 301, 4. Auflage, 1985).

Eine Voraussage, ob ein Hilfsmittel wie DMSO als "Schlepper" für Arzneimittel geeignet ist, ist jedoch nicht möglich und hängt von den Eigenschaften des Wirkstoffs ab (Drug Metabolism Reviews 14 (2), 220 (1983)).

Ein anerkannter Test zur Bestimmung der Wirksamkeit von Antiphlogistika und Antiinflammatorika ist der Test an Entzündungsmodellen (Kaolin-oder Carrageenam-Ödem) bei Rattenpfoten (Jakobi et al, Drug Res. 27, 1326 ff. (1977)), bei dem die Wirkung der Wirkstoffe bestimmt wird. Untersuchungen haben ergeben, daß Arzneimittelzubereitungen aus Etofenamat und DMSO keine Steigerung der Wirkung im Rahmen der Tests an der Rattenpfote zeigen.

Der negative Befund wird bestätigt bei Untersuchungen von anderen handelsüblichen Schleppmitteln, beispielsweise Laurocapran (Azone®) bei Mensch und Tier.

Überraschenderweise wurde jedoch gefunden, daß beim Menschen die Resorption von Etofenamat aus Arzneimitteln, enthaltend Etofenamat und DMSO, erheblich gesteigert wird.

Gegenstand der Erfindung sind Arzneimittelzubereitungen, die Etofenamat und Dimethylsulfoxid enthalten.

In den erfindungsgemäßen Arzneimittelzubereitungen unterstützen sich Etofenamat und DMSO durch signifikante Resorptionssteigerung sowie schnellere Resorption.

Die erfindungsgemäßen Arzneimittelzubereitungen enthalten Etofenamat und DMSO im Gewichtsverhältnis 1:15 bis 1:1, bevorzugt 1:1,7 bis 1:5.

Die erfindungsgemäßen Arzneimittelzubereitungen können in verschiedenen Formen appliziert werden. Bevorzugt sind topische Anwendungen wie Gele, Cremes, Salben, Lotions, Sprays und Lösungen. Insbesondere bevorzugt sind Gele und Cremes.

Erfindungsgemäße gelförmige Arzneimittelzubereitungen enthalten im allgemeinen außer Etofenamat und DMSO, Gelbildner wie Adjuvanzien, Konsistenzgeber und an sich bekannte Zusätze.

Adjuvanzien für die erfindungsgemäßen gelförmigen Arzneimittelzubereitungen können bevorzugt Ester ein-und/oder mehrwertiger Alkohole der Kettenlänge $C_2$ bis $C_{18}$ mit Carbonsäurekomponenten der Kettenlänge $C_6$ bis $C_{18}$ sein.

Alkohol-und Carbonsäurekomponenten sind hierbei im allgemeinen geradkettige oder verzweigte Kohlenwasserstoffreste.

Als Alkoholkomponente seien insbesondere einwertige Alkohole mit 2 bis 6 Kohlenstoffatomen und zwei-und dreiwertige Alkohole mit 2 oder 3 Kohlenstoffatomen genannt.

Als Carbonsäurekomponente seien Carbonsäuren mit einer Kettenlänge von 12 bis 16 Kohlenstoffatomen genannt.

Als Ester seien beispielsweise genannt: Propylenglykoldiester, Ethyloleat, Isopropylmyristat, Isopropylstearat, Isopropylpalmitat, Diisopropyladipat, Di ethylsebacat, Ölsäureoleylester, Laurinsäurehexylester und Isooctylstearat. Insbesondere bevorzugt sind Diisopropyladipat und Isopropylmyristat.

Adjuvanzien für die erfindungsgemäßen Arzneimittelzubereitungen können außerdem bevorzugt höhere Alkohole mit 10 bis 24 Kohlenstoffatomen sein. Der Kohlenwasserstoffrest kann geradkettig oder verzweigt sein. Insbesondere bevorzugt sind höhere Alkohole mit 12 bis 18 Kohlenstoffatomen.

Als höhere Alkohole seien beispielsweise Oleylalkohol und 2-Octyl-dodecanol genannt. Insbesondere bevorzugt ist 2-Octyl-dodecanol.

Arzneimittelzubereitungen können ein oder mehrere, bevorzugt 1 bis 3, Adjuvanzien enthalten.

Als Konsistenzgeber seien beispielsweise genannt: Hochdisperse Kieselsäure, z.B. Aerosil, Hydrogelbildner wie Veegum, Polyglykole, wie Polyethylenglykol und Polypropylenglykol, pharmazeutisch verwendbare Cellulosederivate, wie Methylcellulose, Hydroxyethylcellulose und Carboxymethylcellulose, Carboxyvinylpolymere wie Carbopol® (DE-A 26 41 210), Alginsäurederivate, wie Salze der Alginsäuren und Gummiarabicum.

Als Zusätze seien durchblutungsfördernde oder wärmende Zusätze und Geruchsstoffe genannt.

Durchblutungsfördernde oder wärmende Zusätze können beispielsweise Benzylnicotinat, Salicylsäureester wie Methylsalicylat, etherische Öle, Capsaicin, Capsicumextrakt und Nonylsäurevanillylamid sein.

Als Geruchsstoffe können die üblichen pharmakologisch unbedenklichen Stoffe eingesetzt werden, sofern sie mit den Wirkstoffen und den Adjuvanzien verträglich sind.

Die erfindungsgemäßen gelförmigen Arzneimittelzubereitungen enthalten außerdem noch Wasser und gegebenenfalls niedere Alkohole (geradkettige oder verzweigte oder aromatisch substituierte mit 2 bis 10 Kohlenstoffatomen). Bevorzugt werden Iso-Propanol, Benzylalkohol und Ethanol.

Besonders bevorzugte erfindungsgemäße gelförmige Arzneimittelzubereitungen enthalten im allgemeinen

2 bis 30, bevorzugt 4 bis 12, Gew.-Teile Etofenamat,
10 bis 30, bevorzugt 15 bis 20, Gew.-Teile DMSO,
5 bis 80, bevorzugt 10 bis 40, Gew.-Teile an Adjuvanzien,
0,1 bis 10, bevorzugt 0,5 bis 5, Gew.-Teile Konsistenzgeber,
0,5 bis 5, bevorzugt 1 bis 3,5, Gew.-Teile Zusätze,
15 bis 80, bevorzugt 20 bis 40, Gew.-Teile Wasser und
0 bis 40, bevorzugt 20 bis 35, Gew.-Teile niedere Alkohole.

Die erfindungsgemäßen gelförmigen Arzneimittelzubereitungen können wie folgt hergestellt werden: Etofenamat, DMSO, Adjuvanzien und Zusätze werden in einem Alkohol, beispielsweise Ethyl-oder Isopropylalkohol, gelöst. Anschließend wird der Konsistenzgeber in die Lösung eingerührt. Die Gelbildung wird durch Neutralisation durch eine basische Substanz (beispielsweise Natronlauge) herbeigeführt.

Besonders bevorzugte erfindungsgemäße cremeförmige Arzneimittelzubereitungen enthalten im allgemeinen

20 bis 30, bevorzugt 5 bis 15, Gew.-Teile Etofenamat,
5 bis 30, bevorzugt 10 bis 20, Gew.-Teile DMSO,
5 bis 50, bevorzugt 15 bis 30, Gew.-Teile Adjuvantien,
0 bis 20, bevorzugt 1 bis 8, Gew.-Teile Konsistenzgeber,
0 bis 20, bevorzugt 1 bis 5, Gew.-Teile Emulgatoren,
0,5 bis 5, bevorzugt 1 bis 3,5, Gew.-Teile Zusätze,
30 bis 60, bevorzugt 35 bis 50, Gew.-Teile Wasser und
1 bis 5, bevorzugt 1,5 bis 3, Gew.-Teile niedere Alkohole.

Erfindungsgemäße cremeförmige Arzneimittelzubereitungen enthalten im allgemeinen neben Etofenamat und DMSO noch Cremebildner, Adjuvantien und gegebenenfalls Konsistenzgeber und/oder Emulgatoren und Zusätze wie durchblutungsfördernde bzw. wärmende Stoffe sowie Hilfsstoffe wie z.B. angenehm riechende Stoffe.

Adjuvantien, Konsistenzgeber und Zusätze können im allgemeinen die gleichen Stoffe wie die Gele enthalten.

Als Emulgatoren können einer oder mehrere aus der Gruppe der ionogenen oder nichtionogenen Wasser-in-Öl-und Öl-in-Wasser-Emulgatoren eingesetzt werden.

Bevorzugt werden als Emulgatoren Salze höherer Fett-und Gallensäuren wie Triethanolaminstearat, Alkylsulfate wie Na-Laurylsulfat, Alkylsulfonate wie N-Cetylsulfonat, höhere Fettalkohole wie Cetylalkohol, Laurylalkohol und Stearylalkohol, Sterinalkohole wie Cholesterin, Fettsäureester mehrwertiger Alkohole und Polyole wie Ethylenmonostearat, Glycerinmonooleat, Sorbitanmonolaurat, Sorbitantrioleat, Polyoxyethylen-(20)-sorbitan-monolaurat, Polyoxyethylen-sorbitan-hexaoleat, Fettalkoholether wie Polyoxyethylenlaurylether, Polyoxyethylenoleylether, Fettsäureester der Saccharose wie Saccharosedistearat, Lecithin und Derivate, Betaine und Sulfobetaine wie Fettsäureamidoalkylbetain und/oder Polyoxyethylenpolyoxypropylen-Polymere, wie Pluronics®, eingesetzt.

Die erfindungsgemäßen Creme-Zubereitungen werden hergestellt, indem das Etofenamat und das DMSO mit den übrigen gegebenenfalls aufgeschmolzenen Fett-/Wachs-Emulgator-Bestandteilen gemischt, die Mischung mit der wäßrigen Phase der Zubereitung emulgiert und anschließend einer Homogenisierung beispielsweise mittels einer Rotor-Stator-Einrichtung unterworfen wird.

Die Homogenisierungstemperatur ist von der Zusammensetzung der Zubereitung abhängig und liegt im allgemeinen im Bereich von ca. 35° bis ca. 60°C.

Die erfindungsgemäßen Arzneimittelzubereitungen weisen im Vergleich zu üblichen Etofenamatzubereitungen eine erhebliche Wirkungssteigerung auf. Sie werden daher bevorzugt als Analgetika und Antiphlogistika verwendet.

Beispiel 1

Im randomisierten cross over-Versuch an 12 Probanden wurden signifikante Unterschiede hinsichtlich der Resorption von Etofenamat aus cutan anwendbaren Etofenamat-Zubereitungen, ohne sowie mit DMSO-Anteil, gefunden. Beide Applikationen erfolgten mit gleichen Wirkstoffkonzentrationen und Applikationsmengen. Die Bestimmung der Wirkstoffspiegel im Humanplasma erfolgte mittels HPTLC [B. Beckermann et al., "Proc. 3rd Internat, Symposium on Instrumental High Performance Thin-Layer Chromatography", Würzburg/FRG 1985, Published by Institute for Chromatography, D-6702 Bad Dürkheim/FRG, 15-24 (1985)].
Folgende Unterschiede wurden zwischen den beiden Präparaten gefunden:
-die Zeit maximalen Plasmaspiegels $t_{max}$ nach DMSO-Etofenamat Gel (DEG) im Vergleich zu Etofenamat Gel (EG) signifikant kürzer: 4,2 statt 19 Std. (p < 0,001)
-die maximale Plasmaspiegelkonzentration $c_{max}$ nach DEG doppelt so hoch wie nach EG (p < 0,001)
-die renale Elimination nach DEG in den ersten 12 sowie 24 Std. post applicationem signifikant schneller als nach EG
-die Gesamtresorption, gemessen an der Fläche unter der Konzentrationsleitkurve sowie an der renalen Elimination, nach DEG signifikant höher als nach EG (jeweils p < 0,001) (gepaarter t-Test, L. Sachs, Angewandte Statistik, Seiten 242-243 (1984), Springer Verlag, Berlin, 6. Auflage).

Beispiel 2

Das Resorptionsprofil DMSO-haltiger Etofenamatzubereitungen unterscheidet sich von jenem DMSO-freier Zubereitungen. Es konnte gezeigt werden, daß die schnelle cutane Resorption von Etofenamat in Gegenwart von DMSO ein weitgehend identisches Zeitprofil besitzen, z.B. schneller Anstieg zu Maximalwerten (DMSO nach 5 Stunden, infolge einer lag-time, durch die eine Verzögerungsphase zwischen Applikation und Plasmaspiegel anstieg) und Abfall binnen eines Tages auf niedrige Werte. Im Gegensatz dazu erreicht der DMSO-Metabolit Dimethylsulfon ($DMSO_2$) erst nach über einem Tag Maximalspiegel, welche langsam abfallen.
Die Ergebnisse der DMSO/$DMSO_2$-Kinetik entsprechen den Angaben in K.H. Kolb et al., Ann. N.Y. Acad. Sci. 141, 85-95 (1967) u.a.
Das weitgehend identische Zeitprofil von resorbiertem Antiphlogistikum (Etofenamat) und DMSO ist dagegen neu.
Es beweist, daß DMSO eine positive Schlepperfunktion für Etofenamat besitzt.

Beispiel 3 (Vergleich)

Im Analgesietest nach Randall-Selitto [L.D. Randall and J.J. Selitto, Arch. int. Pharmacodyn. 111, 409-419 (1957)] wurde an männlichen Wistar II-Ratten die Wirksamkeit von handelsüblichen Etofenamat-Gel (5 %ig) sowie 20 % DMSO enthaltendem Etofenamat-Gel vergleichend untersucht. Bei Applikationen von jeweils 50 mg Gel/kg Körpergewicht erwies sich das DMSO-haltige Etofenamat Gel als gleich stark analgetisch wirksam wie die DMSO-freie Zubereitung.
Dies zeigt, daß keine signifikante Steigerung der Etofenamat-Resorption oder Potenzierung der analgetischen Wirkung durch DMSO bei Ratten auftritt.

## Beispiele 4 bis 9 (Gele)

|  | 1 | 2 | 3 | 4 | 5 |
|---|---|---|---|---|---|
| Etofenamat | 5,0 | 5,0 | 5,0 | 5,0 | 5,0 |
| DMSO | 20,0 | 20,0 | 20,0 | 20,0 | 20,0 |
| Diisopropyladipat | 7,5 | 10,0 | - | - | 5,0 |
| Isopropylpyristat | - | - | 5,0 | 7,5 | - |
| Isopropanol | 30,0 | 33,0 | 25,0 | 30,0 | 30,0 |
| etherische Öle | 0,2 | 0,2 | 0,2 | 0,2 | 0,2 |
| Wasser | 26,25 | 26,75 | 29,75 | 23,85 | 30,75 |
| Fettalkoholpolyglykol-ether | 6,0 | - | 6,0 | 6,0 | - |
| Propylethylenglykol 400 | 3,0 | 3,0 | 3,0 | 3,0 | 3,0 |
| Propylenglykol | - | - | 3,0 | 3,0 | 4,0 |
| Hydroxypropylcellulose | - | - | 1,0 | - | - |
| Carboxyvinylpolymer | 1,3 | 1,3 | 1,3 | 1,3 | 1,3 |
| Natronlauge, 10 %ig | 0,75 | 0,75 | 0,75 | - | 0,75 |
| Ammoniaklösung, 28 %ig | - | - | - | 0,15 | - |
|  | 100,0 | 100,0 | 100,0 | 100,0 | 100,0 |

<u>Beispiele 10 bis 14</u> (Creme)

| Beispiel Nr. | 10 | 11 | 12 | 13 | 14 |
|---|---|---|---|---|---|
| Etofenamat | 5,0 | 5,0 | 5,0 | 5,0 | 5,0 |
| DMSO | 10,0 | 10,0 | 20,0 | 20,0 | 20,0 |
| Glycerinmonodistearat | 12,0 | 12,0 | 9,0 | 11,0 | - |
| Glycerinmonostearat | - | - | - | - | - |
| Diisopropyladipat | 13,0 | 13,0 | 16,0 | - | - |
| Isopropyllyristat | - | - | - | 16,0 | 17,0 |
| Cetylalkohol | 3,4 | 3,4 | 3,4 | 1,4 | 3,4 |
| MYRJ 59 | 5,0 | 5,0 | 5,0 | 5,0 | 5,0 |
| Trinatriumcitrat-dihydrat | 0,5 | 0,5 | 0,5 | 0,5 | 0,5 |
| Natriumhydrogencitrat | 0,4 | 0,4 | 0,4 | 0,4 | 0,4 |
| Benzylalkohol | 1,5 | 1,5 | 1,5 | 1,5 | 1,5 |
| Wasser | 49,2 | 49,2 | 39,2 | 39,2 | 37,2 |
| | 100,0 | 100,0 | 100,0 | 100,0 | 100,0 |

**Ansprüche**

1. Arzneimittelzubereitung, enthaltend Etofenamat und Dimethylsulfoxid.

2. Arzneimittelzubereitung nach Anspruch 1, enthaltend Etofenamat und DMSO im Verhältnis 1:15 bis 1:1.

3. Arzneimittelzubereitung nach den Ansprüchen 1 und 2, enthaltend Etofenamat, DMSO und Gelbildner.

4. Arzneimittelzubereitung nach den Ansprüchen 1 und 2, enthaltend Etofenamat, DMSO und Cremebildner.

5. Verfahren zur Herstellung von Arzneimittelzubereitungen von Etofenamat, dadurch gekennzeichnet, daß man Etofenamat und DMSO mit geeigneten Hilfsstoffen zubereitet.

6. Verfahren zur Herstellung von Arzneimittelzubereitungen nach Anspruch 5, dadurch gekennzeichnet, daß man Etofenamat und DMSO im Verhältnis 1:15 bis 1:1 einsetzt.

7. Verfahren nach den Ansprüchen 5 und 6, dadurch gekennzeichnet, daß man Etofenamat und DMSO in einem Alkohol löst, Gelbildner hinzufügt und durch Zugabe einer Base den Gelzustand herstellt.

8. Verfahren nach den Ansprüchen 5 und 6, dadurch gekennzeichnet, daß man Etofenamat und DMSO in einen Cremebildner eingibt und dann homogenisiert.

9. Verwendung von Etofenamat und DMSO zur Herstellung von Analgetika und Antiphlogistika.